# EUROPEAN PATENT SPECIFICATION

(11) **EP 2 427 125 B1**
(45) Date of publication and mention of the grant of the patent: **11.04.2018**
(21) Application number: 10721315.9
(22) Date of filing: 10.05.2010
(51) Int. Cl.: A61B 17/06

(54) **THREAD HAVING COATED ANCHORING STRUCTURES FOR ANCHORING IN BIOLOGICAL TISSUES**
FADEN MIT BESCHICHTETEN VERANKERUNGSSTRUKTUREN ZUR VERANKERUNG IN BIOLOGISCHEN GEWEBEN
FIL PRÉSENTANT DES STRUCTURES D'ANCRAGE REVÊTUES POUR ANCRAGE DANS DES TISSUS BIOLOGIQUES

(30) Priority: 08.05.2009 DE 102009020897
(43) Date of publication of application: 14.03.2012
(73) Proprietor: Aesculap AG, 78532 Tuttlingen/Donau (DE); ITV Denkendorf Produktservice GmbH, 73770 Denkendorf (DE)
(72) Inventor: ODERMATT, Erich, 8200 Schaffhausen (CH); BERNDT, Ingo, 78532 Tuttlingen (DE); MÜLLER, Erhard, 70565 Stuttgart (DE); OBERHOFFNER, Sven, 71384 Weinstadt-Endersbach (DE)
(74) Representative: Patentanwälte Ruff, Wilhelm, Beier, Dauster & Partner mbB
(86) International application number: PCT/EP2010/002846
(87) International publication number: WO 2010/127873

(56) References cited:
- WO-A2-2009/042841
- US-A- 5 222 976
- US-A1- 2005 267 532

## Description

The present invention relates to a thread, more particularly in the manner of a knotless or self-fixating suture. Surgeons generally close wounds using thread-shaped sutures. These are typically knotted to achieve secure fixation in the tissue. Here care must be taken to ensure that the wounds to be closed are stitched together using an optimal force at the wound edges. If, for example, the wound edges are stitched together too loosely and too nonuniformly, there is a risk in principle of increased scarring or dehiscence. If, by contrast, the wound edges are stitched together overly tautly, there is a risk that blood flow through the wound edges is restricted, which can give rise to necrotic changes in the surrounding tissue region.

In addition to the risk of possible secondary complications, which may necessitate renewed surgical interventions, there is also always a certain risk that wound closures based on knotted sutures will lead to disruptions in the healing process and more particularly to unsatisfactory cosmeses for the patients concerned. Another factor is that it is often necessary for several knots, more particularly up to seven knots, to be placed on top of each other to ensure a secure knotted hold. This means that there is a lot of material being introduced into the region of the wound to be cared for, and can lead to increased foreign-body reactions, more particularly in the case of absorbable sutures. Furthermore, the use of conventional sutures can also be of disadvantage in the management of difficult-to-access fields of indication, as typically occur in laparoscopy.

Sutures which, in contradistinction to known or traditional threads, do not have to be knotted have already been known for some time under the designation "barbed sutures". Such knotless or self-locking/self-fixating/self-retaining sutures usually consist of a monofil thread that has barbs along its longitudinal axis. Corresponding sutures are described for example in the printed publications US 3,123,077 A, EP 1 559 266 B1, EP 1 560 683 B1 and EP 1 556 946 B1. The barbs have generally been formed on the thread surface such that the thread can be pulled through the tissue in the direction of the barbs without great resistance and more particularly without significant tissue traumas. When pulled in the opposite direction, however, the barbs deploy and anchor themselves and hence also the suture in a surrounding tissue region. This prevents the suture being pulled back through a puncture channel formed by the suture.

It is a profound disadvantage that the barbs are generally fine structures having an intrinsic softness and hence flexural slackness. Flexurally slack barbs can lead to problems in respect of surgical utility particularly in the case of soft and more particularly elastomeric thread materials. This can impair the ability of the barbs to "drill" into a biological tissue. Secondly, an intrinsic flexural slackness on the part of the barbs heightens the profound risk that the barbs fold over under stresses arising in the body of a patient and as a result thereof the thread material will slip through the puncture channel formed in the course of implantation of the suture.

Document WO 2009/042841 discloses self-retaining sutures including tissue retainers having improved strength, according to the preamble of independent claim 1.

It is an object of the present invention to provide a thread, more particularly in the manner of a knotless or self-fixating suture, that provides very secure tissue fixation and/or tissue lifting.

We have found that this object is achieved according to the present invention by a thread having the features of independent claim 1. Preferred embodiments of the thread are subject matter of dependent claims 2 to 12. The present invention further provides a wound closure system having the features of independent claim 13 and also a surgical kit of parts having the features of independent claim 14. The thread of the present invention comprises a thread, more particularly in the manner of a knotless or self-fixating suture. The thread comprises an elongate thread body having on its surface protruding anchoring structures for anchoring in biological, more particularly human and/or animal, tissues, wherein the elongate thread body and the anchoring structures are completely covered by a stiffening coating.

The invention thus provides a thread useful in surgery (a surgical thread) whose anchoring structures are in a stiffened state due to a coating. The coating provided according to the present invention increases with particular advantage the flexural stiffness of the anchoring structures. This improves their ability to "drill" or penetrate into a surrounding region of tissue. Moreover, the stiffened anchoring structures are less prone to folding over after implantation of the thread, minimizing the risk of the implanted thread slipping out of the puncture channel. Altogether, therefore, the thread of the present invention is more firmly and hence more securely anchorable in a biological tissue than conventional threads of the same type.

Biological tissues for the purposes of the present invention may in principle comprise hard and/or soft tissues. The tissues are preferably selected from the group consisting of skin, fat, fascia, bone, muscles, organs, nerves, blood vessels, connective tissue, sinews, tendons and/or ligaments. The thread of the present invention is generally useful for tissue fixation, more particularly for surgical closure of a wound, and/or for tissue lifting. The thread is more particularly suitable for applications in the field of plastic surgery, preferably therein for tightening the skin, and cheek and/or jaw line correction. For example, the thread is useful for performing an eyebrow lift. In addition, however, the thread of the present invention is also suitable for other surgical indications, more particularly for indications where the use of conventional sutures is made difficult by steric hindrance for example. The thread can also be used with particular advantage for performing laparoscopic interventions. A further field of use concerns the fixation of implants, more particularly textile implants, preferably medical meshes. Examples of textile implants include hernia, prolapse or urinary incontinence meshes. A further possible field of application for the thread of the present invention relates to the performance of anastomoses, more particularly vascular or intestinal anastomoses.

In a further embodiment, the anchoring structures are uniformly surrounded or covered by the stiffening coating. In other words, the stiffening coating is preferably formed uniformly on the anchoring structures. Uniformly coated anchoring structures are generally notable for optimal hardness and hence stiffness, more particularly flexural stiffness, and therefore this kind of anchoring structures is particularly capable of "drilling" into a biological tissue. In addition, uniformly coated anchoring structures are particularly less prone to the risk of folding over as a result of loading forces arising in the body of a patient.

The stiffening coating is also formed on the surface of the elongate thread body. According to the present invention, more particularly, the elongate thread body is covered completely, i.e. including the anchoring structures, by the stiffening coating.

In a possible embodiment, the stiffening coating is formed uniformly on the elongate thread body and uniformly on the anchoring structures protruding therefrom.

In general, the coated anchoring structures protrude from the surface of the thread body.

In a further suitable embodiment, the stiffening coating comprises between 0.5% and 25% by weight, more particularly 1% and 20% by weight, preferably 2% and 15% by weight, based on the total weight of the thread (elongate thread body plus anchoring structures including the stiffening coating). The stiffening coating preferably has a layer thickness between 1 and 100 µm, more particularly 2 and 50 µm, preferably 4 and 40 µm.

In a particularly preferred embodiment, the stiffening coating comprises a material, preferably a polymer, having a flexural modulus > 1500 N/mm², preferably > 4000 N/mm². Preferably, the stiffening coating is formed of such a material, more particularly polymer.

In principle, the stiffening coating may comprise the same material, more particularly be formed of the same material, as the elongate thread body and the anchoring structures. However, it is preferable according to the present invention for the stiffening coating to comprise a different material, more particularly be formed of a different material, than the elongate thread body and the anchoring structures.

The stiffening coating advantageously comprises a biocompatible material, preferably a biocompatible polymer. More particularly, the stiffening coating is formed of a biocompatible material, preferably polymer. The polymer may comprise a homopolymer or a copolymer. Copolymer herein is to be understood as meaning a polymer composed of two or more different monomeric units. According to the present invention, therefore, the biocompatible polymer may comprise for example a terpolymer or tetrapolymer. Copolymers useful according to the present invention may be selected from the group consisting of random copolymers, gradient copolymers, alternating or turn-taking copolymers, block copolymers, more particularly multi- or oligoblock copolymers, for example diblock, triblock or tetrablock copolymers, segmented copolymers and combinations, more particularly blends, thereof.

In an advanced embodiment, the stiffening coating is absorbable, partially absorbable or nonabsorbable. In a preferred embodiment, the stiffening coating comprises a biocompatible, absorbable polymer. Preferably, the polymer comprises a polyhydroxyalkanoate, preferably from the group consisting of polylactide, polyglycolide, poly-ε-caprolactone, polytrimethylene carbonate, poly-p-dioxanone, copolymers thereof and combinations, more particularly blends, thereof.

In an alternative embodiment, the stiffening coating comprises a biocompatible, nonabsorbable polymer. The polymer is preferably selected from the group consisting of polyethylene terephthalate, polypropylene terephthalate, polybutylene terephthalate, polypropylene, polyvinylidene difluoride, polytetrafluoroethylene, polytetrafluoropropylene, poly-hexafluoropropylene, polyamides, copolymers thereof and combinations, more particularly blends, thereof.

According to the present invention, furthermore, the stiffening coating may be formed of one of the polymers, copolymers or polymer combinations, more particularly blends, mentioned in the previous embodiments.

In principle, the elongate thread body and the anchoring structures may comprise different materials, more particularly different polymers, or be formed of different materials, more particularly different polymers. Preferably, the elongate thread body and the anchoring structures comprise alike materials, more particularly alike polymers, or are formed of alike materials, more particularly alike polymers.

The thread body and the anchoring structures may in principle be formed of all materials, more particularly polymeric materials, suitable for this. The thread body and/or the anchoring structures may be absorbable, partially absorbable or nonabsorbable. The polymers may comprise homopolymers or copolymers, more particularly ter- or tetrapolymers. Copolymers may more particularly be selected from the group consisting of random copolymers, gradient copolymers, alternating or turn-taking copolymers, block copolymers, more particularly multi- or oligoblock copolymers, for example diblock, triblock or tetrablock copolymers, segmented copolymers and combinations, more particularly blends, thereof.

The thread body and/or the anchoring structures are preferably formed of an absorbable, partially absorbable or nonabsorbable polymer. Examples of absorbable polymers can be selected from the group consisting of polylactide, polyglycolide, poly-ε-caprolactone, poly-p-dioxanone, polytrimethylene carbonate, poly-3-hydroxybutyrate, poly-4-hydroxybutyrate, copolymers thereof and combinations, more particularly blends, thereof. In an advanced embodiment, the thread body and/or the anchoring structures are formed of an absorbable co- or terpolymer comprising at least monomeric units from the group consisting of lactide, glycolide, trimethylene carbonate, p-dioxanone, ε-caprolactone, poly-3-hydroxybutyrate, poly-4-hydroxybutyrate and combinations thereof. For example, the thread body and/or the anchoring structures may be formed of a terpolymer, preferably triblock terpolymer comprising glycolide, trimethylene carbonate and ε-caprolactone.

In an alternative embodiment, the thread body and/or the anchoring structures are formed of a nonabsorbable polymer. The nonabsorbable polymer may be selected from the group consisting of polyolefins, polyesters, polyamides, polyurethanes, copolymers thereof and combinations, more particularly blends, thereof. For example, the nonabsorbable polymer may be selected from the group consisting of polypropylene, polyethylene terephthalate, polypropylene terephthalate, polybutylene terephthalate, polytetrafluoroethylene, polytetrafluoropropylene, poly-hexafluoropropylene, nylon, copolymers thereof and combinations, more particularly blends, thereof.

More particularly, the thread body and/or the anchoring structures may be formed of an elastomer. The elastomer is preferably selected from the group consisting of thermoplastic elastomers based on olefin, thermoplastic elastomers based on urethane or thermoplastic polyurethanes, thermoplastic copolyesters, styrene block copolymers, thermoplastic copolyamides, silicone elastomers, copolymers thereof and combinations, more particularly blends, thereof. The thermoplastic elastomers based on urethane, or thermoplastic urethanes, preferably comprise polyurethanes selected from the group consisting of aliphatic polycarbonate urethanes, aromatic polycarbonate urethanes, silicone polycarbonate urethanes, silicone polyether urethanes and combinations, more particularly blends, thereof.

In a further embodiment, the elongate thread body and the anchoring structures are formed in one piece. More particularly, as already mentioned, the anchoring structures may be formed of an alike material, more particularly of an alike polymer, to the thread body. The anchoring structures are preferably formed as cuts into the surface of the elongate thread body. The cuts may comprise mechanical, physico-chemical, more particularly laser-produced or thermal cuts. The cuts may have a cut depth, measured perpendicularly to the surface of the elongate thread body, of between 5 and 60%, more particularly 15 and 50%, preferably 20 and 40%, based on the diameter of the thread body.

In a further possible embodiment, the anchoring structures are moulded onto the surface of the elongate thread body. The moulding of the anchoring structures can take place during the production of the thread body, for example through injection moulding, or in a subsequent processing step.

The anchoring structures may form a barb, escutcheon, shield, scale, wedge, spike, arrow, v and/or w shape on the surface of the elongate thread body. It is particularly preferable according to the present invention when the anchoring structures form a barb shape, or barbs, on the surface of the elongate thread body.

Furthermore, the anchoring structures may in principle form different arrangements on the surface of the elongate thread body. For example, the anchoring structures may form a linear arrangement, a row-shaped arrangement, an offset arrangement, a zigzag-shaped arrangement, a spiral-shaped arrangement, a helical-shaped arrangement, a random arrangement or combinations thereof on the surface of the elongate thread body, more particularly in its longitudinal and/or transverse direction, preferably in its longitudinal direction. A spiral-shaped arrangement of the anchoring structures on the surface of the elongate thread body provides particularly secure and firm anchoring of the thread in a biological tissue and therefore is preferred.

In a further embodiment, the elongate thread body displays on its surface at least one set, more particularly two, three or more sets, of anchoring structures. A set of anchoring structures is herein to be understood as meaning an arrangement of anchoring structures on the surface of the thread body which in respect of the configuration of the anchoring structures, for example in respect of the length of the anchoring structures, height of the anchoring structures, cut depth of the anchoring structures, angle of the anchoring structures formed by the anchoring structures with the surface of the thread body, orientation of the anchoring structures and/or shape or form of the anchoring structures coincides.

In a further possible embodiment, the anchoring structures form a unidirectional arrangement on the surface of the thread body. In other words, the anchoring structures in this embodiment point uniformly in one direction.

Preferably, however, the thread displays a so-called bidirectional arrangement of anchoring structures on the surface of the elongate thread body. A bidirectional arrangement of anchoring structures is herein to be understood as meaning an arrangement wherein the anchoring structures are formed in two different directions (bidirectionally) on the surface of the thread body. Preferably, when viewed in the longitudinal direction of the thread body, the anchoring structures for a first portion of the thread body point in the direction of a remaining second portion of the thread body and for the remaining second portion of the thread body point in the direction of the first portion of the thread body. For example, viewed in the longitudinal direction of the thread body, the anchoring structures for a first portion of the thread body can be formed to point in the direction of the thread middle and for a remaining second portion of the thread body can be formed also to point in the direction of the thread middle. Here the length of the thread-body portions can correspond approximately to half the thread-body length.

In an advanced embodiment, the thread displays on the surface of its thread body at least two bidirectional arrangements of anchoring structures. It is particularly preferable when, relative to a first bidirectional arrangement of anchoring structures, a second bidirectional arrangement of anchoring structures is formed on the surface of the thread body in the circumferential direction of the thread body by about 180° and preferably offset to the first bidirectional arrangement. It can further be provided according to the present invention for the thread to display three bidirectional arrangements of anchoring structures on the surface of its thread body. It is preferable in this case when, relative to a first bidirectional arrangement of anchoring structures, a second bidirectional arrangement of anchoring structures is formed on the surface of the thread body in the circumferential direction of the thread body by about 120° and preferably offset to the first bidirectional arrangement, and this second bidirectional arrangement of anchoring structures is in turn formed in the circumferential direction of the thread body by about 120° and preferably offset, relative to a third bidirectional arrangement of anchoring structures, so that the third bidirectional arrangement of anchoring structures is similarly formed in the circumferential direction of the thread body by about 120° and preferably offset relative to the first bidirectional arrangement of anchoring structures.

In a further preferred embodiment, the anchoring structures have a periodically changing, more particularly alternating, orientation on the surface of the elongate thread body. For example, viewed in the longitudinal direction of the thread body, the anchoring structures for a first portion of the thread body can be formed in the direction of a second portion of the thread body and for the second portion of the thread body can be formed in the direction of the first portion of the thread body, for a third portion of the thread body adjoining the second portion of the thread body in the direction of a fourth portion of the thread body, and for the fourth portion of the thread body in the direction of the third portion of the thread body, and so on.

It can further be provided according to the present invention for the elongate thread body to display areal regions or areal portions without anchoring structures. Preferably, the thread body displays an areal portion without anchoring structures approximately in the region of the thread middle. This areal portion, viewed in the longitudinal direction of the thread, can have a length between 0.5 and 5 cm, more particularly 1.5 and 3 cm. This makes it possible for example to place the thread ends side by side to form a loop and to attach them to a surgical inserting instrument, preferably to a surgical needle. Preferably, in this embodiment, the remaining areal portions of the thread body display a bidirectional arrangement of anchoring structures, so that, after loop formation, the anchoring structures point unidirectionally in the direction of the loop. Since the thread middle is more particularly used for closing a wound gap, a thread body whose thread middle displays an areal portion without anchoring structures can be used with particular advantage to avoid additional irritations of tissue in the wound region. With regard to the possible arrangements for anchoring structures on the surface of the thread body, the description heretofore is referenced in its entirety.

According to the present invention, the thread can be formed as a monofilament or as a multifilament, in particular as a braided or intertwined multifilament. The thread is preferably formed as a monofilament. In principle, the thread of the present invention may also be formed as a pseudomonofilament.

In a further embodiment, the thread is a mass or solid thread. This preferably means that the thread has no lumen. In particular, the thread body may be designed as a mass or solid thread body.

In a further embodiment, the thread is designed as a hollow thread, in particular as a tubular thread, preferably as a tube or a hose. Further, it is preferred that the hollow thread has a closed wall. Besides, it is preferred that the hollow thread comprises open ends. By way of example, a hollow thread may be produced by means of extrusion. The anchoring structures may protrude into the interior and/or exterior of the hollow thread. A protrusion of the anchoring structures into the exterior of the hollow thread is preferred. The anchoring structures may further be present as cuts into the wall of the hollow thread, wherein the cuts preferably do not break through the wall of the hollow thread. As an alternative or in combination, the anchoring structures may be present as breakthroughs, i.e. the anchoring structures are formed completely breaking through the wall of the hollow thread. Such hollow threads may be used for various medicinal, in particular surgical, applications. For instance, hollow threads according to the present invention may be used as self-anchoring infusion tubes, delivery tubes, catheters, distribution systems for pharmaceutical agents, in particular liquid pharmaceutical agents, drug-release-systems, wherein the drugs are preferably stored within a lumen of the hollow thread, or drainage systems, in particular drainage tubes. Further, hollow threads according to the present invention may be applied, in particular may be shrunk and/or drawn, on a round stock, fibre, monofilament, pseudomonofilament, multifilament, thread, yarn or the like in order to equip these structures with self-anchoring properties.

In particular, the thread body may be designed as a hollow thread body, in particular as a tubular thread body, preferably as a tube or hose. With respect to further details and advantages, reference is made in its entirety to the previous paragraph.

The thread generally has a circular cross section. However, other cross-sectional shapes are conceivable as well. For example, the thread may have an oval, triangular, trilobal, square, trapezoidal, rhomboid, pentagonal/five-cornered, hexagonal/six-cornered, star-shaped or cruciform cross section. Such cross-sectional shapes are readily realizable with the aid of appropriate extrusion dies which can be custom made to any desired cross-sectional shape.

In a further embodiment, the thread, preferably the stiffening coating, includes additives, more particularly biological and/or therapeutic actives. For example, the thread, preferably the stiffening coating, may include actives selected from the group consisting of antimicrobial actives, disinfecting actives, growth-promoting actives, anti-inflammatory actives, blood-coagulating actives, pain-killing (analgesic) actives, odour-controlling actives and combinations thereof.

In a further embodiment, the thread is present in the form of a continuous-filament thread or as a converted, more particularly cut-to-length, thread.

In a further advantageous embodiment, the thread is connected at least at one end, preferably at both ends, to a surgical inserting instrument. The surgical inserting instrument preferably comprises a surgical needle. The use of a canula as inserting instrument is similarly possible according to the present invention. Preferably, at least one end of the canula has been sharpened with an oblique grind in order that a small cut be made on penetration into a biological tissue. Alternatively, the canula may also have a conically tapered and more particularly pointed end. In the case of the above-described loop-shaped thread, it is generally the case that both ends of the thread are attached to a surgical inserting instrument. However, when the thread has a bidirectional arrangement of anchoring structures on the surface of the thread body, it is preferable according to the present invention when both of the ends of the thread are each connected to a surgical inserting instrument. To connect the thread to a surgical inserting instrument, the thread can be inserted into a dedicated hole in the inserting instrument and the inserting instrument subsequently be compressed or beaded in the region of the hole.

To avoid puncture channel bleeds, it can be further provided according to the present invention for the thread to have a smaller diameter in the region of its ends than in the remaining thread regions. In other words, the ends of the thread may have a tapered diameter. Such a thread is particularly advantageously combined with a surgical inserting instrument, for example a surgical needle, that is actually designed for a smaller thread diameter. In this way, the thread diameter can be conformed to the diameter of the inserting instrument. According to the present invention, especially a diameter ratio of surgical inserting instrument to thread < 2:1, preferably of 1:1, can be provided. As a result, a puncture channel formed by the surgical inserting instrument can be better filled out by the thread regions having the original, i.e. untapered, diameter. A puncture channel whose diameter is very close to the diameter of the thread (without anchoring structures) is advantageous in contributing to better anchoring of the thread in the tissue. To taper the diameter, the thread can be shaved in the region of its ends. Thermal methods or laser techniques can be used for this for example. The transition from the original diameter of the thread to the tapered diameter in the region of the thread ends can be abrupt or continuous, more particularly in the form of a gradient. To form a gradual transition, extrusion technology is suitable in particular. The take-off speed involved in extruding a thread can be varied, more particularly periodically. This can be accomplished for example by modulating the circumferential speed of the godet responsible for the take-off of the thread. Alternatively, additional godets can be interposed between the extrusion die and the take-off godet.

In a further embodiment, the thread is present in a drawn or undrawn state. Generally, however, the thread is present in a drawn state.

A further aspect of the present invention relates to a wound closure system or a surgical fixating means, more particularly in the manner of a knotless or self-fixating surgical suture, comprising a thread as per the present invention. The wound closure system is generally present as a thread-shaped wound closure system. As such, the wound closure system is preferably formed to be monofil. In respect of further features and details concerning the wound closure system, reference is made to the description heretofore.

The present invention further provides a surgical kit of parts, more particularly for knotless or self-fixating tissue fixation or tissue lifting, comprising a thread or a wound closure system as per the present invention and at least one surgical inserting instrument, preferably two surgical inserting instruments. As already mentioned, the surgical inserting instrument may comprise a surgical needle or a canula. With regard to further features and details concerning the kit of parts, reference is likewise made to the description heretofore.

The invention further discloses an exemplary process for producing a thread, a thread comprising an elongate thread body having on its surface protruding anchoring structures for anchoring in biological, more particularly human and/or animal, tissues has a stiffening coating formed at least partially, more particularly completely or uniformly, at least on the anchoring structures. In general, the stiffening coating is formed on the anchoring structures such that an embedding of the anchoring structures into the coating to form a smooth, more particularly pseudo-smooth, surface of the thread does not take place.

The stiffening coating can be formed using for example dipping techniques, spraying techniques, wipe-off techniques, for example by means of a long-haired brush, or extrusion techniques, more particularly a sheathing extrusion. When the stiffening coating is formed by means of sheathing extrusion, the anchoring structures are sensibly not formed on the thread-body surface until after the sheathing extrusion. Further ways to provide, preferably cover or coat, the anchoring structures with a stiffening coating, consist in depositing an appropriate coating material from the gas phase.

In an alternative exemplary process of producing a thread, the anchoring structures are cut into the surface of the elongate thread body. For example, the anchoring structures can be cut into the thread body mechanically, more particularly by means of at least one cutting blade, preferably a microtome knife or, as an alternative, by means of a needle, in particular a surgical needle, in order to less affect the mechanical stability of the thread body. Customary cutting devices can be used for this purpose. Useful cutting devices generally comprise a cutting board, at least one cutting blade and also holding or fixing elements for the thread body, for example a vice, chucks, holding or clamping jaws. For example, cutting the anchoring elements mechanically may utilize a cutting board (cutting abutment) having a groove, the groove being intended to receive the thread body to be cut into. Depending on the depth of the groove, the use of at least one cutting blade allows specific control of the cut depth to which the anchoring structures are cut into the thread body. This is because the at least one cutting blade is generally configured such that with it only at most the regions of the thread body which protrude from the groove can be cut into. This makes it possible to avoid inadvertently cutting too deeply into the thread body, which would compromise the strength of the thread.

In an alternative exemplary process of producing a thread, the anchoring structures are cut into the elongate thread body thermally, preferably in a temperature range between 10 and 100°C, more particularly 20 and 50°C, above the melting point of the thread material. Thermal cutting of the anchoring structures has the profound advantage over mechanical cutting that the cut ends in the thread body which are produced by thermal cutting are less tapered, more particularly less acute, than results from purely mechanical cutting.

This can be used to minimize the risk of the thread developing, under load, a tear starting from the respective cut ends. In an advanced embodiment, the anchoring structures are cut into the thread body by means of a cutting wire, more particularly metal wire, suitable for this purpose. A heated, more particularly an electrically heated, cutting wire can be used for example. The cutting wire preferably comprises a fine wire. Preference is given to using a cutting wire having a diameter between 20 and 50 µm. As an alternative to a single cutting wire, it is also possible to use a sheet of cutting wires. It is similarly possible according to the present invention to use a metal wire grid or wire grid for cutting the anchoring structures into the thread body.

In a further alternative exemplary process of producing a thread, the anchoring structures are cut into the elongate thread body by means of laser cutting techniques. Useful lasers include in principle not only gas lasers, for example CO₂ lasers, but also solid state lasers, for example Nd:YAG lasers. In general, a suitable laser cutting machine consists of a laser beam source, a beam guide and a usually mobile system of focusing optics (concave mirror or positive lens). The beam leaving the beam source is guided either through an optical fibre in the case of an Nd:YAG laser for example, or via a deflecting mirror in the case of a CO₂ laser for example, to the machining optics which focus the laser beam and thereby produce the power densities required for cutting, which generally range from 10⁶ to 10⁹ W/cm². Appropriate laser cutting processes are well known to a person skilled in the art, so that more far-reaching observations can be dispensed with here.

The cutting of the anchoring structures can be effected in the drawn or undrawn state of the elongate thread body. Any drawing is preferably effected while heat is applied. To produce a heat advantageous for the drawing operation, hot air, steam, warm water or infrared radiation can be used for example. It is similarly possible for drawing to be carried out in a heating oven suitable for this purpose. To draw the thread body, it is usually guided over a roller or godet system comprising a set of rollers or godets which can have different speeds of rotation. Generally, each subsequent roller has a higher speed of rotation than the preceding roller of the drawing system. However, alternatively to the just-described continuous drawing, it is also possible to carry out discontinuous drawing. For discontinuous drawing, the thread body can be clamped for example between the clamping jaws of a clamping device and subsequently drawn.

After drawing, optionally also independently of any drawing, of the thread it can be envisaged for the thread to be subjected to various post-treatment steps. In general, the thread is for this heat-conditioned in vacuo or at reduced pressure. This can be used to raise the crystallinity of the thread and, more particularly, lower a residual monomer content. A further advantage which can be achieved through a post-treatment of the thread relates to the reduced susceptibility to shrinking.

In a further exemplary process of producing a thread, the elongate thread body is subjected to a rel-exation, in particular shrinkage, subsequent to a drawing. Thus, the righting angle of the anchoring structures may be minimized. This is in particular advantageous with respect to polymers having a high drawing ratio. Such polymers may otherwise contribute to anchoring structures that may protrude basically perpendicular from the thread body. Further, due to a relaxation, elasticity of the thread may be improved. This leads to improved handling properties and, in particular to a more equal transfer of force on tissue to be treated.

In a further exemplary process of producing a thread, the elongate thread body is subjected to a rel-exation, in particular shrinkage. Afterwards, the anchoring structures are cut into the relaxed thread body. Subsequently, the cut-in thread body is drawn. For example, a monofilament made of polypropylene may be relaxed to about 50 % up to about 70 % in relation to its originally length. Preferably, the relaxation of a monofilament made of polypropylene is performed at a temperature of about 160 °C. Afterwards, the anchoring structures are cut into the polypropylene thread. Thereafter, the cut-in polypropylene thread is drawn, in particular using a drawing factor between 1.2 and 2.0. The exemplary process described in this paragraph also facilitates a minimization of the righting angle of the anchoring structures.

The present invention also provides for the use of the thread of the present invention for producing a wound closure system or a surgical fixating means, more particularly in the manner of a knotless or self-fixating surgical suture. The wound closure system or surgical fixating means is particularly useful for tissue fixation, preferably for a surgical closure of a wound. A further field of application relates to tissue lifting, more particularly skin lifting, in the field of plastic surgery. Further fields of application relate to the abovementioned cheek and/or jaw line correction. With regard to further features and details, the description heretofore is referenced in its entirety.

Finally, the present invention also provides for the use of the thread of the present invention for producing a surgical fixating means for implants, more particularly textile implants, preferably meshes. The meshes may comprise for example hernia meshes, urinary incontinence meshes or prolapse meshes. With regard to further features and details, the description heretofore is similarly referenced in its entirety.

Further features of the invention will become apparent from the following description of preferred embodiments by reference to two examples and figure descriptions in conjunction with the features of the subclaims and the figures. Individual features here can each be actualized on their own or two or more at a time in combination with each other.
Figure 1 is a schematic drawing of an embodiment of the inventive thread,
Figure 2 is a schematic drawing of an exemplary thread, and
Figure 3 is a schematic drawing of a further exemplary thread.

### 1. Examples

### 1.1 Reference example

An undrawn monofile spinning thread was produced from a block terpolymer consisting of 72 % by weight of glycolide, 14 % by weight of trimethylenecarbonate and 14 % by weight of caprolactone, each related to the total weight of the polymer (trade name monosyn). The production was made by means of thermoplastic extrusion. The thread was tempered for one hour at 80 °C in order to increase its crystallinity. Afterwards, 50 cuts were made into the undrawn spinning thread by means of a cutting apparatus. The cuts were made in such a way that the cutting angle was 25°, the cutting depth was 29 % in relation to the thread's diameter, and the cutting distance was 0,28 mm. Further, the monofile thread was rotated under an angle of 120° around the longitudinal axis of the thread, in order to form a helix-shaped arrangement of the cuts. Subsequently, the cut-in thread was drawn to the fourfold of its original length in a slit heater at a temperature of 140 °C. Thus, protruding barbs were formed. The produced self-anchoring monofile thread had a linear tensile strength of 32,1 N, an elongation at break of 58 % and a diameter in thread areas where no barbs were present of 0,49 mm. The flexural modulus of the thread only amounted to about 450 N/mm². Thus, the thread was extremely flexible and pliable.

The produced thread was attached at one end thereof to a G21-cannula (without luer-lock), in order to investigate the pull-out strength of the thread with respect to animal tissue. The attachment to the G21-cannula was done in such a manner that the barbs were orientated away from the needle. Thus, it was ensured that the barbs did not block when the thread was pulled into the tissue (swine abdomen with callosity, thickness about 2 cm). After pulling the thread into the tissue, a pull-out strength of 3,8 N was measured by means of a tensile testing machine.

### 1.2 Example with a stiffening coating

For coating the barbs of a thread as produced according to example 1.1, a 15 % by weight solution of a LDL7030 polymer (copolymer of 70 % poly-L-lactide and 30 % of poly-DL-lactide) in dichloromethane was provided. Afterwards, the thread, which is insoluble in dichloromethane, was clamped in a rotating device and directly placed above a tray that was completely filled with the LDL7030 polymer solution. The thread was placed above the tray in such a way that basically only the protruding barbs were in contact with the polymer solution. Next, the thread was rotated around its longitudinal axis during a time period of 30 seconds, so as to ensure that the barbs were equally coated with a polymer solution. Immediately, afterwards, the thread (under further rotation) was placed in an oven, in order to evaporate dichloromethane at 120 °C. After two minutes, the thread was removed from the oven. It turned out that the thread was predominantly coated with the LDL7030 polymer in the area of the barbs. In other words, basically merely the barbs were coated with the LDL7030 polymer.

Due to the coating (stiffening) of the barbs, the pull-out strength of the thread in view of swine abdomen with a callosity was increased up to 6,7 N. The tensile strength (31,9 N) and the elongation at break (53 %) remained nearly unaffected. Further, the bending stiffness of the monofile thread was also not basically increased, since the coating was predominantly present on the barbs.

### 2. Figure description

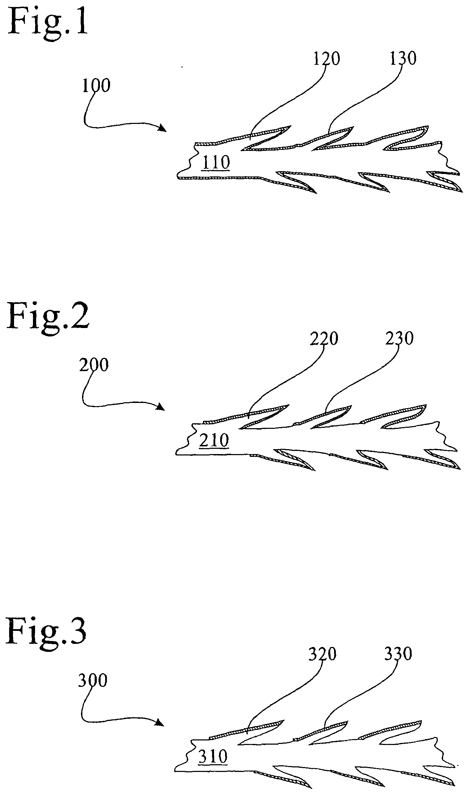

Figure 1 depicts an embodiment of an inventive thread 100. The thread 100 has an elongate thread body 110 having anchoring structures 120 formed on its surface, preferably in the manner of barbs. The anchoring structures 120 serve to provide knotless or self-fixating anchoring of the thread 100 in a biological tissue. The tissue in question generally comprises the tissue of a human or animal patient. The anchoring of the thread 100 is the result of the anchoring structures 120 "drilling" or penetrating into the surrounding tissue. The surface of the thread body 110 is covered completely, i.e. inclusive of the surfaces of the anchoring structures 120, by a stiffening coating 130. The layer thickness of the stiffening coating 130 is generally chosen such that the coated anchoring structures 120 are still discernible as structures protruding from the surface of the thread body 110. The coating 130 endows the anchoring structures 120 with stiffer, more particularly flexurally stiffer, properties. As a result of the increased stiffness of the anchoring structures 120, it is simpler for them to come into contact with a surrounding region of tissue and achieve better "drilling" or penetration into a tissue. In addition, the stiffened anchoring structures 120 are less prone to folding over after the thread 100 has been placed in a biological tissue in the manner of a knotless or self-fixating suture. The reduced susceptibility of the coated anchoring structures 120 to folding over in vivo reduces the risk that the thread 100 will slip out of the puncture channel and thereby for example undesirably loosen a surgical closure of a wound or a tissue lifting.

Figure 2 shows an exemplary thread 200. The thread 200 has an elongate thread body 210 having anchoring structures 220 protruding from its surface, preferably in the manner of barbs. In contradistinction to the embodiment depicted in Figure 1, the exemplary thread 200 has a stiffening coating 230 formed on the anchoring structures 220 only. Elsewhere, the surface of the thread body 210 is essentially free of the stiffening coating 230. With regard to further features and details reference is made as far as possible to the figure description relating to Figure 1 in its entirety.

Figure 3 depicts a further exemplary thread 300, which likewise has an elongate thread body 310. Again, the surface of the thread body 310 displays anchoring structures 320 protruding therefrom. However, in the exemplary thread depicted in Figure 3 it is merely the backs of the anchoring structures 320 that are covered with a stiffening coating 330. Elsewhere, the surface of the thread body 310 is free of the stiffening coating 330. With regard to further features and details reference is made as far as possible to the figure description relating to Figure 1 in its entirety.

## Claims

1. Thread (100), more particularly in the manner of a knotless or self-fixating suture, comprising an elongate thread body (110) having on its surface protruding anchoring structures (120) for anchoring in biological, more particularly human and/or animal, tissues, wherein the anchoring structures (120) have a stiffening coating (130), **characterized in that** the elongate thread body (110) and the anchoring structures (120) are completely covered by the stiffening coating (130) and the completely coated anchoring structures (120) protrude from the surface of the thread body (110).

2. Thread (100) according to claim 1, **characterized in that** the stiffening coating (130) is formed uniformly on the anchoring structures (120).

3. Thread (100) according to claim 1 or 2, **characterized in that** the stiffening coating (130) comprises between 0.5% and 25% by weight, more particularly 1% and 20% by weight, preferably 2% and 15% by weight, based on the total weight of the thread (100).

4. Thread (100) according to any preceding claim, **characterized in that** the stiffening coating (130) is formed of a different material than the elongate thread body (110) and the anchoring structures (120).

5. Thread (100) according to any preceding claim, **characterized in that** the stiffening coating (130) comprises a biocompatible polymer, more particularly is formed of a biocompatible polymer.

6. Thread (100) according to any preceding claim, **characterized in that** the stiffening coating (130) comprises an absorbable polymer, more particularly selected from the group consisting of polylactide, polyglycolide, poly-ε-caprolactone, polytrimethylene carbonate, poly-para-dioxanone, copolymers thereof and combinations thereof.

7. Thread (100) according to any preceding claim, **characterized in that** the stiffening coating (130) comprises a nonabsorbable polymer, more particularly selected from the group consisting of polyethylene terephthalate, polybutylene terephthalate, polypropylene, polyvinylidene difluoride, polytetrafluoroethylene, polyhexafluoropropylene, polyamides, copolymers thereof and combinations thereof.

8. Thread (100) according to any preceding claim, **characterized in that** the anchoring structures (120) form a barb, escutcheon, shield, scale, wedge, spike, arrow, v and/or w shape, preferably a barb shape, or barbs, on the surface of the elongate thread body (110).

9. Thread (100) according to any preceding claim, **characterized in that** the anchoring structures (120) form a linear arrangement, row-shaped arrangement, offset arrangement, zigzag-shaped arrangement, spiral-shaped arrangement, helical-shaped arrangement, random arrangement or combinations thereof on the surface of the elongate thread body (110).

10. Thread (100) according to any preceding claim, **characterized in that** the anchoring structures (120) form a uni- or bidirectional arrangement, preferably a bidirectional arrangement, on the surface of the elongate thread body (110).

11. Thread (100) according to any preceding claim, **characterized in that** the anchoring structures (120) have a periodically changing, more particularly alternating, orientation on the surface of the elongate thread body (110).

12. Thread (100) according to any preceding claim, **characterized in that** the thread is a mass thread, or is designed as a hollow thread, in particular as a tubular thread, preferably as a tube.

13. Wound closure system, more particularly in the manner of a knotless or self-fixating suture, comprising a thread (100) according to any preceding claim.

14. Surgical kit of parts, more particularly for knotless or self-fixating tissue fixation or tissue gathering, comprising a thread (100) according to any one of claims 1 to 12 or a wound closure system according to claim 13 and at least one surgical inserting instrument.

## Patentansprüche

1. Faden (100), insbesondere nach Art eines knotenlosen bzw. selbstfixierenden Nahtmaterials, umfassend einen länglichen Fadenkörper (110), auf dessen Oberfläche davon abstehende Verankerungsstrukturen (120) zur Verankerung in biologischen, insbesondere menschlichen und/oder tierischen, Geweben ausgebildet sind, wobei die Verankerungsstrukturen (120) eine versteifende Beschichtung (130) aufweisen,
**dadurch gekennzeichnet, dass**
die versteifende Beschichtung (130) vollflächig auf dem länglichen Fadenkörper (110) und den Verankerungsstrukturen (120) ausgebildet ist und die vollflächig beschichteten Verankerungsstrukturen (120) von der Oberfläche des Fadenkörpers (110) abstehen.

2. Faden (100) nach Anspruch 1, **dadurch gekennzeichnet, dass** die versteifende Beschichtung (130) auf den Verankerungsstrukturen (120) gleichmäßig ausgebildet ist.

3. Faden (100) nach Anspruch 1 oder 2, **dadurch gekennzeichnet, dass** die versteifende Beschichtung (130) einen Anteil zwischen 0,5 und 25 Gew.-%, insbesondere 1 und 20 Gew.-%, vorzugsweise 2 und 15 Gew.-%, bezogen auf das Gesamtgewicht des Fadens (100), aufweist.

4. Faden (100) nach einem der vorhergehenden Ansprüche, **dadurch gekennzeichnet, dass** die versteifende Beschichtung (130) aus einem anderen Material gebildet ist als der längliche Fadenkörper (110) und die Verankerungsstrukturen (120).

5. Faden (100) nach einem der vorhergehenden Ansprüche, **dadurch gekennzeichnet, dass** die versteifende Beschichtung (130) ein bioverträgliches Polymer aufweist, insbesondere aus einem solchen gebildet ist.

6. Faden (100) nach einem der vorhergehenden Ansprüche, **dadurch gekennzeichnet, dass** die versteifende Beschichtung (130) ein resorbierbares Polymer, insbsondere ausgewählt aus der Gruppe bestehend aus Polylactid, Polyglykolid, Poly-ε-Caprolacton, Polytrimethylencarbonat, Poly-para-dioxanon, Copolymere davon und Kombinationen davon, aufweist.

7. Faden (100) nach einem der vorhergehenden Ansprüche, **dadurch gekennzeichnet, dass** die versteifende Beschichtung (130) ein nicht resorbierbares Polymer, insbesondere ausgewählt aus der Gruppe bestehend aus Polyethylenterephthalat, Polybutylenterephthalat, Polypropylen, Polyvinylidendifluorid, Polytetrafluorethylen, Polyhexafluorpropylen, Polyamide, Copolymere davon und Kombinationen davon, aufweist.

8. Faden (100) nach einem der vorhergehenden Ansprüche, **dadurch gekennzeichnet, dass** die Verankerungsstrukturen (120) widerhaken-, wappen-, schild-, schuppen-, keil-, stachel-, pfeil-, v- und/oder w-förmig, vorzugsweise widerhakenförmig bzw. nach Art von Widerhaken, auf der Oberfläche des länglichen Fadenkörpers (110) ausgebildet sind.

9. Faden (100) nach einem der vorhergehenden Ansprüche, **dadurch gekennzeichnet, dass** die Verankerungsstrukturen (120) in einer linearen Anordnung, reihenförmigen Anordnung, versetzten Anordnung, zickzackförmigen Anordnung, spiralförmigen Anordnung, schraubenförmigen Anordnung, zufälligen Anordnung oder in Kombinationen davon auf der Oberfläche des länglichen Fadenkörpers (110) ausgebildet sind.

10. Faden (100) nach einem der vorhergehenden Ansprüche, **dadurch gekennzeichnet, dass** die Verankerungsstrukturen (120) uni- oder bidirektional, vorzugsweise bidirektional, angeordnet auf der Oberfläche des länglichen Fadenkörpers (110) ausgebildet sind.

11. Faden (100) nach einem der vorhergehenden Ansprüche, **dadurch gekennzeichnet, dass** die Verankerungsstrukturen (120) auf der Oberfläche des länglichen Fadenkörpers (110) eine sich periodisch ändernde, insbesondere alternierende, Ausrichtung besitzen.

12. Faden (100) nach einem der vorhergehenden Ansprüche, **dadurch gekennzeichnet, dass** der Faden in Form eines massiven Fadens vorliegt oder als Hohlfaden, insbesondere als schlauchartiger Faden, bevorzugt als Schlauch gestaltet ist.

13. Wundverschlusssystem, insbesondere nach Art eines knotenlosen oder selbstfixierenden Nahtmaterials, umfassend einen Faden (100) nach einem der vorhergehenden Ansprüche.

14. Chirurgisches Kit mit Teilen, insbesondere zur knotenlosen oder selbstfixierenden Gewebefixierung oder Geweberaffung, umfassend einen Faden (100) nach einem der Ansprüche 1 bis 12 oder ein Wundverschlusssystem nach Anspruch 13 und zumindest ein chirurgisches Einführinstrument.

## Revendications

1. Fil (100), en particulier sous forme d'un matériel de suture sans noeud ou auto-fixatrice, comprenant un corps de fil allongé (110) présentant sur sa surface des structures d'ancrage protubérantes (120) pour ancrage dans des tissus biologiques, en particulier des tissus humains et/ou animaux, les structures d'ancrage (120) présentant un revêtement raidissant (130),
**caractérisé en ce que**
le corps de fil allongé (110) et les structures d'ancrage (120) sont recouverts complètement par le revêtement raidissant (130) et les structures d'ancrage (120) complètement revêtues dépassent de la surface du corps de fil (110).

2. Fil (100) selon la revendication 1, **caractérisé en ce que** le revêtement raidissant (130) est formé uniformément sur les structures d'ancrage (120).

3. Fil (100) selon la revendication 1 ou 2, **caractérisé en ce que** le revêtement raidissant (130) compose entre 0,5 % and 25 % en poids, en particulier 1 % et 20 % en poids, de préférence 2 % et 15 % en poids, par rapport au poids total du fil (100).

4. Fil (100) selon l'une des revendications précédentes, **caractérisé en ce que** le revêtement raidissant (130) est formé d'un matériau différant du matériau du corps de fil allongé (110) et des structures d'ancrage (120).

5. Fil (100) selon l'une des revendications précédentes, **caractérisé en ce que** le revêtement raidissant (130) comprend un polymère biocompatible, en particulier est formé d'un polymère biocompatible.

6. Fil (100) selon l'une des revendications précédentes, **caractérisé en ce que** le revêtement raidissant (130) comprend un polymère résorbable, en particulier sélectionné parmi le groupe constitué par polylactide, polyglycolide, poly-ε-caprolactone, poly(carbonate de triméthylène), poly-para-dioxanone, leurs copolymères et leurs combinaisons.

7. Fil (100) selon l'une des revendications précédentes, **caractérisé en ce que** le revêtement raidissant (130) comprend un polymère non-résorbable, en particulier sélectionné parmi le groupe constitué par poly(téréphtalate d'éthylène), poly(téréphtalate de butylène), polypropylène, poly(difluorure de vinylidène), polytétrafluoroéthylène, polyhexafluoropropylène, polyamides, leurs copolymères et leurs combinaisons.

8. Fil (100) selon l'une des revendications précédentes, **caractérisé en ce que** les structures d'ancrage (120) forment un barbillon, un écusson, un bouclier, une écaille, une cale, une pointe, une flèche, une forme de v et/ou de w, de préférence une forme de barbillon, ou des barbillons sur la surface du corps de fil allongé (110).

9. Fil (100) selon l'une des revendications précédentes, **caractérisé en ce que** les structures d'ancrage (120) forment un arrangement linéaire, un arrangement sous forme rangée, un arrangement sous forme décalée, un arrangement sous forme zigzag, un arrangement sous forme de spirale, un arrangement sous forme hélicoïdale, un arrangement aléatoire ou leurs combinaisons sur la surface du corps de fil allongé (110).

10. Fil (100) selon l'une des revendications précédentes, **caractérisé en ce que** les structures d'ancrage (120) forment un arrangement unidirectionnel ou bidirectionnel, de préférence un arrangement bidirectionnel, sur la surface du corps de fil allongé (110).

11. Fil (100) selon l'une des revendications précédentes, **caractérisé en ce que** les structures d'ancrage (120) présentent une orientation changeant périodiquement, en particulier alternant, sur la surface du corps de fil allongé (110).

12. Fil (100) selon l'une des revendications précédentes, **caractérisé en ce que** le fil est un fil de masse, ou est conçu sous forme de fil creux, en particulier sous forme de fil tubulaire, de préférence sous forme de tube.

13. Système de fermeture de plaie, en particulier de la manière d'un matériel de suture sans noeud ou auto-fixatrice, comprenant un fil (100) selon l'une des revendications précédentes.

14. Trousse chirurgicale de composants, en particulier pour fixation de tissu ou rassemblement de tissu sans noeud ou auto-fixatrice, comprenant un fil (100) selon l'une des revendications 1 à 12 ou un système de fermeture de plaie selon la revendication 13 et au moins un instrument chirurgical d'insertion.
